# EUROPEAN PATENT APPLICATION

(11) **EP 4 403 126 A1**
(43) Date of publication of application: **24.07.2024**
(21) Application number: 23152905.8
(22) Date of filing: 23.01.2023
(51) Int. Cl.: A61B 34/10, A61B 34/20, A61B 90/00, A61F 2/38

(54) **PATELLA-TRACKING FOR DETERMINATION OF ROTATIONAL ALIGNMENT OF "A" TIBIAIMPLANT**

(71) Applicant: Aesculap AG, 78532 Tuttlingen (DE)
(72) Inventor: UTZ, Michael, 78532 Tuttlingen (DE); DUPRAZ, Ingrid, 78532 Tuttlingen (DE); LARRAINZAR-GARIJO, Ricardo, 28007 Madrid (ES)
(74) Representative: Winter, Brandl - Partnerschaft mbB

(57) **Abstract**

The present disclosure relates to a system and method for providing alignment and positioning data of an endoprosthesis fitting of joints, performing the steps of:
- acquiring information as input data on the position and course of the native trochlea of a first bone in relation to the joint centre, the joint line of the first bone and the joint line of a second bone forming the joint together with the first bone, and the bony axes of the first and second bones;
- processing the acquired input data and calculating output data to provide alignment and positioning data of a first joint partial implant of the first bone having an artificial trochlea and a second joint partial implant of the second bone, wherein the artificial trochlea of the first joint partial implant is to replace the native trochlea of the first bone as identically as possible in alignment and position;
- providing the output data as alignment and positioning data.

## Description

### Technical field

The invention relates to a pre- and/or intra-operative method for providing alignment and positioning data of implant components in the endoprosthesis fitting of joints, in particular knee joints.

### Background of the invention

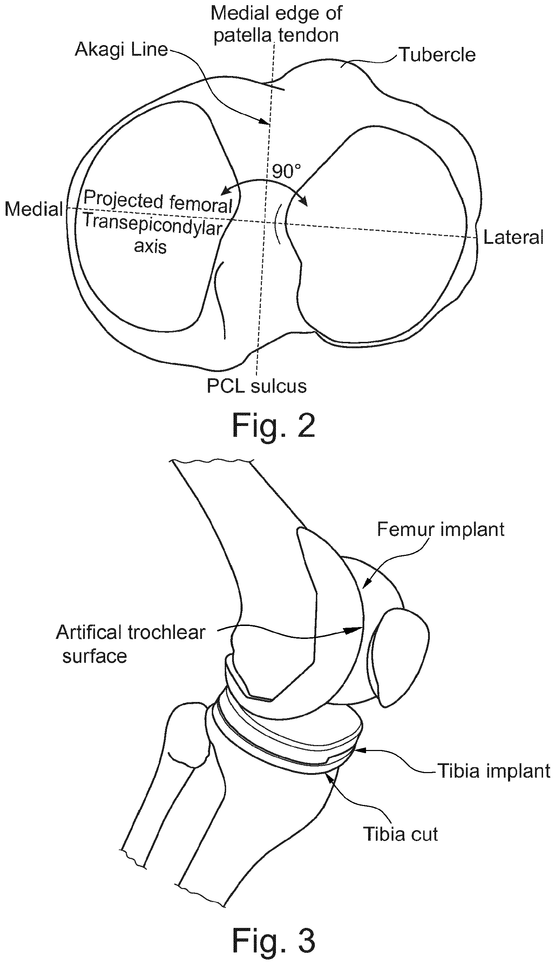

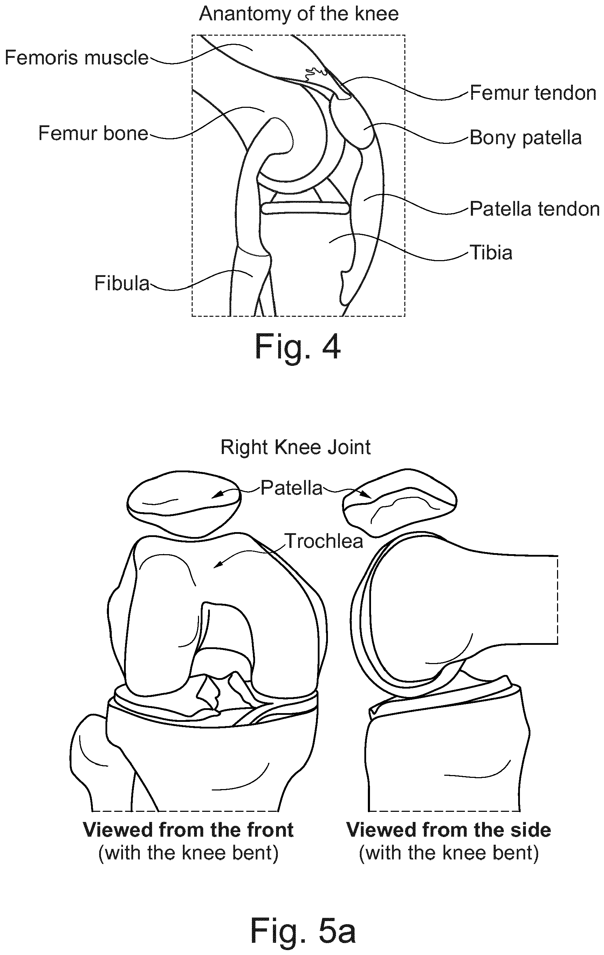

Surgical procedures are already known from the prior art that rely on the use of single, usually geometric, parameters in determining an optimal alignment and positioning of an implant for the patient. A concrete example of this is the rotational alignment of a tibial implant during total knee arthroplasty (TKA) as shown, for example, in Fig.3, which is usually determined and performed using the antero-posterior axis of the tibial plateau. A commonly used definition of this axis of the tibial plateau is the so-called Akagi Line (see Fig. 2), which runs from the attachment of the posterior cruciate ligament to the medial third of the tuberosity. Often, a slight rotation to this axis as indicated in Fig. 2 is performed to better compensate for the removal of a bone cover due to the tibial incision for the implant. In some cases, the attachment of the patellar tendon (see Fig. 4) is also used as a guide for alignment and positioning.

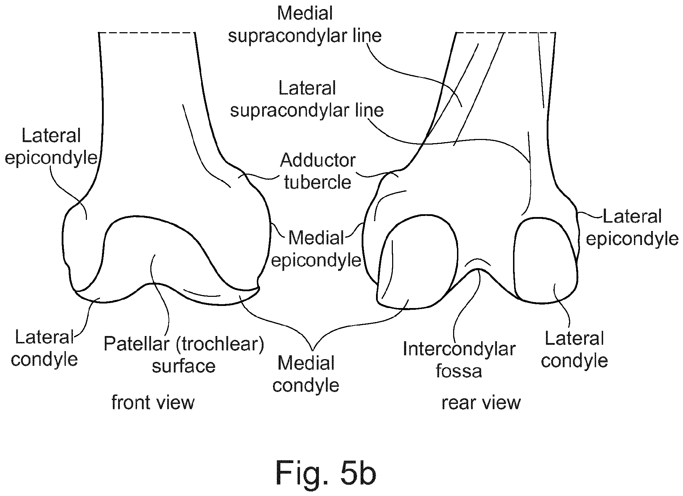

It is also known from the prior art that the rotational alignment of the tibia has a major influence on the force vector of the patella / patella tendon, its change in direction and size over flexion and extension of the knee joint. Nevertheless, to date, little consideration has been given to this for intra-operative alignment of the tibial component, as the clinician has no information on the position of the trochlea which is shown, for example, in Fig. 5a and 5b.

Accordingly, the state of the art has the disadvantage that the force vector of the patella /patella tendon, its change in direction and magnitude over flexion and extension of the knee joint, and thus the run of the patella / patella tendon in the trochlea are usually not taken into account. Nor is the position and orientation of the femoral implant as it is shown in Fig. 3, and thus the femoral trochlea, to the overall knee implant considered.

The patellar tendon runs from the tibial tuberosity to the quadriceps femoris muscle and thus spans between the tibia and femur as it is shown in Fig. 4. Embedded in it is the bony patella, which, during extension and flexion movements of the knee joint, is guided in the femoral trochlea. Compressive forces between the patella and femur can be more than 4 times the body weight. Excessive compressive forces can lead to retropatellar pain or patellar fracture, an unfavourable force vector to patellar dislocation.

Thus, both the position and orientation of the tibial implant and the femoral implant as shown in Fig. 3 directly affect the postoperative patellar compressive forces, their magnitude and direction, and the amount of tension in the patellar tendon. Therefore, both the position and orientation of the tibial implant play a crucial role in the success of endoprosthesis fittings of the knee.

Most importantly, the medio-lateral positioning of the femur is related to the medio-lateral position of the tibia (see Fig. 2), as there is a form-fit between these two components under load, and affects the direction of the patellar force vector. This is because the femoral implant also displaces the femoral trochlea medio-laterally, thus affecting the vector of compressive forces.

The proximo-distal, as well as the ante-posterior position of the femoral implant, in turn, have an influence on the magnitude of the force vector because they increase or reduce the tension in the patellar tendon. Likewise, the proximo-distal position of the tibial implant.

The direction and magnitude of the force vector indirectly reflect the likelihood of patellar dislocation or patellar pain, and thus patient satisfaction. Incorrect positioning of the tibial implant can negatively influence the course of the patella in the trochlea of the femoral implant due to the frictional connection with the femoral implant under load and the resulting forced position and alignment of the femoral implant.

### Summary of the invention

The aim of the invention is that the native course of the femoral trochlea, and thus the soft tissue situation and the force vector of the patella, are recorded pre- or intra-operatively and taken into account for the positioning and orientation of the tibia component.

In other words, it is the aim of the invention to optimize the position and alignment of the trochlea on the femoral implant, as well as the position and alignment of the tibial implant, such that not only the femoro-tibial kinematics of the joint, but also the femoro-patellar kinematics contribute to a "Forgotten Joint". The term "forgotten joint" is meant to express that the patient forgets the joint as a result of a successful implant treatment and thus does not perceive the implant as disturbing, but instead perceives it as a normal/ native, healthy joint.

It is therefore the object of the invention to avoid or at least mitigate the disadvantages of the prior art.

This object is solved by a system and method according to the disclosure in that a system and method for providing alignment and positioning data of an endoprosthesis fitting of joints comprise the following features / method steps:
- an acquiring unit (input interface) being adapted to acquire information as input data on the position and course of the native trochlea of a first bone in relation to the joint centre, to the joint line of the first bone and to the joint line of a second bone forming the joint together with the first bone, and to the bony axes of the first and second bones;
- a processing unit (CPU) being adapted to process the acquired input data and calculating output data to provide alignment and positioning data of a first joint partial implant of the first bone having an artificial trochlea and a second joint partial implant of the second bone, wherein the artificial trochlea of the first joint partial implant is to replace the native trochlea of the first bone in alignment and position as identically as possible and
- an output or provisioning unit (output interface) being adapted to output or provide the output data as alignment and positioning data.

The aforementioned system and method have the advantage of taking into account a previously unused plurality of patient parameters such as the course of the native trochlea of a first bone in relation to a joint centre, to a joint line of the first bone and to a joint line of a second bone as well as to the bony axes of the first and second bones. Consideration of the aforementioned multitude of different patient parameters, all of which have significant influence on the functioning of a joint being treated, significantly increases the success of joint implant treatments.

Computer-aided processing of the multitude of parameters by the processing unit makes it possible to evaluate the multitude of parameters and to provide the surgeon with a treatment recommendation in real time based on the evaluated parameters, either before the implant treatment or even during the implant treatment. The computer-aided analysis and processing makes it possible to evaluate a relatively large number of parameters quickly and to provide a treatment recommendation. The interdependencies of the respective parameters are also taken into account during the analysis. This computer-aided approach to preparing or performing an operation relieves the workload of the surgeon, since a large number of parameters are already suggested as treatment recommendations, and also supports the surgeon, since effects or corrections during an operation are also analyzed and incorporated into a real-time treatment
recommendation.

Further advantageous embodiments are claimed in the subclaims and are explained in more detail below.

An advantageous embodiment is characterized in that the input data refers to a knee joint according to which the first bone is a femur with a femoral trochlea and the second bone is a tibia.

Precisely because the knee joint is subjected to relatively high loads due to its position in the body, it is all the more important to be able to ensure alignment and positioning of an artificial knee joint that is as patient-friendly as possible.

For the knee in particular, the above information can be used from the comprehensive parameter analysis to observe the position and course of the native femoral trochlea in relation to the knee centre, the femoral and tibial joint line and the bony axes before and/or during an endoprosthesis fitting of the knee joint, but at least before the first bony incision. During arthroplasty, this information helps to align both the femoral and tibial implants together so that the trochlea of the femoral implant restores the native trochlea in position and alignment as well as possible.

Furthermore, it is preferred that the system and method for providing alignment and positioning data of an endoprosthesis fitting of joints further can comprise the following features / steps:
- said acquiring unit is adapted to simultaneously consider (and optionally acquire) measured input data of a patellar ligament tension during alignment (alignment procedure) of the tibia;
- said processing unit is adapted to process and match the patellar ligament tension input data with reference values and
- said output or provisioning unit is adapted to provide output data for tibial alignment based on predetermined target tolerance ranges of ligament stress.

The above features and procedural steps are advantageous precisely because intra-operative displacements of the femoral implant are often necessary in order to be able to respond to the pathological situation in the native joint and the associated ligament situation. Thanks to the aforementioned features and procedural steps, the influence of these displacements on the femoro-patellar joint can now be displayed.

According to an advantageous further development of the system and method for providing alignment and positioning data of an endoprosthesis fitting of joints the system and method can comprise the following features and steps:
- said processing unit is adapted to apply best-fit algorithms before and/or during surgery to calculate a position and alignment for the femoral and tibial implant based on at least one preselected parameter of the input data and processing the same into output data and
- said output or provisioning unit is adapted for real-time provision of the output data for the alignment and positioning of the femoral and tibial implant, wherein the provided alignment and positioning enable the individual best possible kinematics of the knee joint for the respective patient.

According to another advantageous development of the system and method for providing alignment and positioning data of an endoprosthesis fitting of joints the best-fit algorithms can consider over the complete range of motion of the knee joint at least one of the following parameters:
- tension of the knee ligament apparatus, preferably including the patellar tendon;
- position of a joint line of the femur and/ or tibia connected to the joint;
- forces expected to result in the knee implant (femoral and tibial);
- pressure expected between the patella implant and the femoral trochlea;
- magnitude and direction of the patellar tendon force vector;
- position of the patella in the trochlea.

One advantage of using the best-fit method is that a large number of selectable parameters are taken into account over the entire range of motion of the knee, so that an alignment and positioning of the implant components can be determined that allows the best possible kinematic interaction of the implant components for all positions of the knee joint in the range of motion of the knee joint. It is precisely this global consideration of the individual parameters over the entire range of motion of the knee that increases the probability of a successful treatment for the patient and that the patient perceives the implant as a "forgotten joint" in his post-operative everyday life and does not perceive it as disturbing or restricting.

In this way, the individual implant components, such as the femoral implant and the tibial implant, can theoretically be ideally aligned and errors are less likely than with previously used alignment methods.

In another preferred embodiment of the system and method for providing alignment and positioning data of an endoprosthesis fitting of joints, results of an intra-operative joint gap measurement can be taken into account in the best-fit algorithms.

Furthermore, it is preferred that in the system and method for providing alignment and positioning data of an endoprosthesis fitting of joints, pre-operative kinematic data, preferably also in the form of gait analyses, can be taken into account in the best-fit algorithms.

The consideration of data from gait analysis in particular can provide conclusions about pathological phenomena in the knee that may not be identified as clearly by simply looking at individual parameters, such as the ligamentous apparatus of the knee, and thus may not be able to be adequately corrected. By taking gait analysis into account, it is therefore possible to ensure a highly individualized prosthetic fitting for the patient.

A further advantageous embodiment of the system and method for providing alignment and positioning data of an endoprosthesis fitting of joints is characterized in that the best-fit algorithms take into account and process the influence of changes to the alignment and orientation of the implant components in the course of a pre-operative and/or intra-operative planning and/or implementation in the best-fit calculations in the form of input data in real time and subsequently reproduce the effects on the individual aspects as output data.

An advantage of the above embodiment of the system and method is that the results of the calculations from the best-fit algorithms are already available to the surgeon as part of a pre-operative planning and that the surgeon can be shown the respective changes for different treatment scenarios. This embodiment of the system and method also makes it possible to react during surgery, intra-operatively, to unforeseen events, for example deviations from the pre-operative analysis in the joint or in the connected joint periphery, or to the occurrence of unexpected interference factors that require an adjustment of the endoprosthesis fitting, in that the best-fit algorithms recalculate the changed parameters and make a correspondingly adjusted suggestion to the surgeon.

Furthermore, it is also appropriate that the system and method for providing alignment and positioning data of an endoprosthesis fitting of joints can be applied to an elbow joint and that the first bone is a humerus and the second bone is an antebrachium.

According to an advantageous further development of the system and method of providing alignment and positioning data of an endoprosthesis fitting of joints, which is designed for an elbow joint can comprise the following features and steps:
- said processing unit is adapted to apply best-fit algorithms before and/or during surgery to calculate a position and alignment for the humeral and antebrachial implant based on at least one preselected parameter of the input data and processing the same into output data and
- said output or provisioning unit is adapted for real-time provision of the output data for the alignment and the positioning of the humeral and antebrachial implant, wherein the provided alignment and positioning enable the individual best possible kinematics of the elbow joint for the respective patient.

### Brief description of the drawings

The present disclosure is explained in more detail below with the aid of the following figures:
Fig. 1 is a flow diagram for the selection of a particular joint and the subsequent application of a method for providing alignment and positioning data of an endoprosthesis fitting of joints,
Fig. 2 is a top view of a human tibia,
Fig. 3 is a perspective view of an artificial knee (knee implant),
Fig. 4 is a side view of a human knee,
Fig. 5a is a front and side view of a bent human knee explicitly showing the trochlea,
Fig. 5b is a front and rear view of the distal end of a human femur;
Fig. 5c is a front (a) and rear (b) view of a human humerus.

### Description of embodiments

Examples of embodiments of the present disclosure are described below on the basis of the accompanying figures. The same elements are indicated by the same reference signs. Features of the individual embodiments may be interchanged with one another.

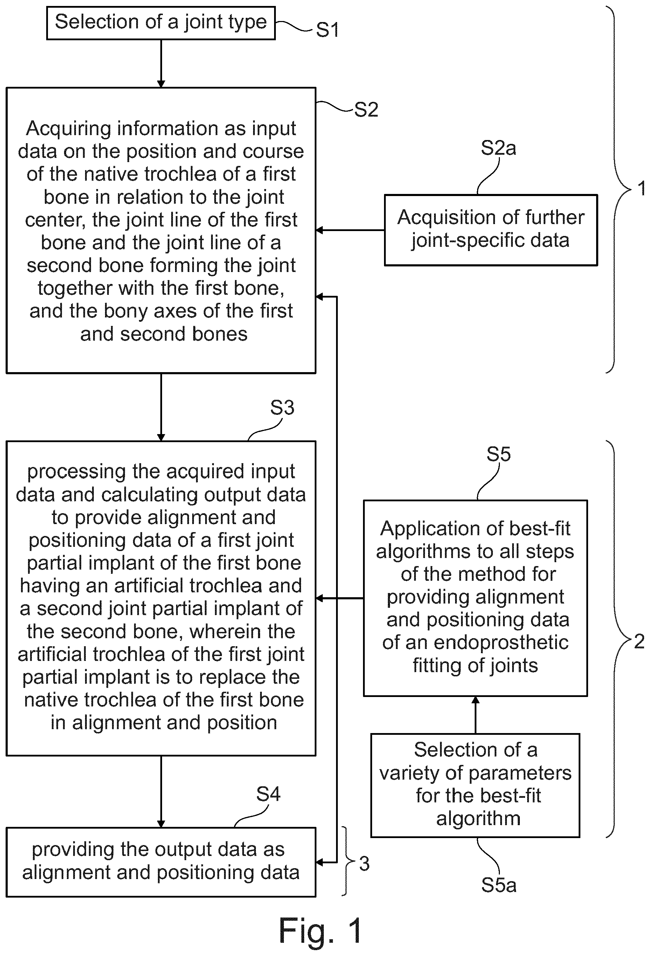

Fig. 1 shows a flow diagram of a modular procedure being processed by a processing system according to the present disclosure for endoprosthesis fitting of a joint. The system and method is intended to assist clinical personnel in planning endoprosthesis fittings for a patient and/or during intra-operative endoprosthesis fitting in order to align and position an artificial joint implant in a way that is as patient-friendly as possible, i.e. adapted to the individual physical conditions of a patient. In other words, the system and method are adapted for providing alignment and positioning data in order to align and position joint implant components in a manner that is as patient-specific as possible.

The system and method for providing alignment and positioning data for endoprosthesis fittings of joints can in principle be applied to all types of joints. However, the embodiments of the system and method described below refer in particular to embodiments for providing alignment and positioning data for endoprosthesis fittings of knee joints.

### First embodiment of a system and method providing alignment and positioning data of an endoprosthesis fitting of joints

As shown in Fig. 1, clinical personnel, for example a surgeon, first selects in step 1 (S1) of the flow diagram, the type of joint to be endoprosthetically treated using an input interface 1, for example in form of a terminal, touch screen or the like, wherein general joint information may be pre-stored in a data base (not shown). Then, a procedure for providing alignment and positioning data for endoprosthesis fitting being adapted to the selected type of joint can be applied to that specific joint, hereinafter the knee joint by a processing unit (CPU) 2.

In this regard, the method / procedure for providing alignment and positioning data for endoprosthesis fitting may be stored and retrievable on any data processing unit / device, such as a computer, tablet, or other type of data processing devices. Moreover, the method/ procedure for providing data for endoprosthesis fitting may also be stored in any sort of cloud service and the aforementioned cloud service, for example, can be accessed via an access-protected web front end (with authorization constraint) and used via the web front end. The selection of the respective procedure for the respective joint is made via any kind of input interfaces as already mentioned above and the procedure results can be provided/output/displayed on any kind of output interface like a display that is either wired or wirelessly data-connected to the processing unit on which the procedure is preferably located.

In step 2 (S2) the actual method/process for providing alignment and positioning data for endoprosthesis fitting is started by acquiring information as input data on the position and course of the native trochlea of a first bone in relation to the joint centre, the joint line of the first bone and the joint line of a second bone forming the joint together with the first bone, and the bony axes of the first and second bones. In the application of the method/process to the knee, the first bone is a femur with femoral trochlea and the second bone is a tibia.

The input data used may come from various measurement techniques, in particular imaging techniques such as ultrasound techniques, computed tomography (CT) techniques, magnetic resonance imaging (MRI) techniques, or positron emission tomogroaphic (PET) techniques as further input interfaces, commonly representing the acquiring unit 1, and may be passed to the data processing device used. Furthermore, the input data can also come from wireless positioning sensors such as inertial measurement unit (IMU) or optical navigation systems.

After the data is acquired in step 2 (S2) by the acquiring unit 1, it is processed in step 3 (S3) by the data processing device 2 used and calculations to provide output data of alignment and positioning data of a first partial femoral knee joint implant, which has an artificial femoral trochlea and a second partial knee joint implant of the tibia, wherein the artificial femoral trochlea of the first partial knee joint implant is to replace the native trochlea of the first femur as identically as possible in alignment and position.

Finally, in step 4 (S4), the output data are provided by an output interface 3 to the surgeon, in real time, as alignment and positioning data. The alignment and positioning data can be displayed in the form of 2D or 3D representations of the knee joint and its adjacent periphery. The representation can be displayed by the output interface for example on an output device such as a display, VR glasses, a tablet or similar device. Virtual resection planes as well as their angular offset and (height) position relative to orientation axes or leg axes (i.e., for example, the longitudinal axis of the femur or tibia) can also be superimposed in the representations. This shows the surgeon where to place a resection on the femur and tibia for implant placement based on the processed input data. The placement and alignment of the individual partial implants on the femur and tibia can also be displayed virtually.

A particular advantage of the system and method described is that it is suitable for preoperative planning and allows various surgical scenarios with different input data to be planned through before clinical staff agree on a preferred surgical scenario after consultation.

But also intra-operatively, the aforementioned method offers great advantages, because even in the case of unexpected complications during a surgical operation and thus to a changed input data situation can be reacted in real time. Thanks to real-time data processing, it is possible to determine a new best possible treatment option for the unexpected change during the operation.

### Second embodiment of a method providing alignment and positioning data of an endoprosthesis fitting of joints

The second embodiment of a system and method providing alignment and positioning data of an endoprosthesis fitting of joints is mostly identical to the first embodiment of the system and method and differs only in step 2a (S2a).

In the second embodiment, additional joint-specific information is acquired as input data in step 2a (S2a). Specifically for the knee joint, the patellar ligament tension is acquired during the alignment of the tibia.

Subsequently, this data is also processed in step 3 (S3) and compared with ligament tension reference values from a database, and then output data is provided in step S4 (S4), which takes into account predefined target tolerance ranges of the ligament tension during the alignment of the tibia.

### Third embodiment of a system and method providing alignment and positioning data of an endoprosthesis fitting of joints

The third embodiment of a system and method providing alignment and positioning data of an endoprosthesis fitting of joints is also mostly identical to the first embodiment of the system and method but differs in step 5 (S5) and step 5a (S5a).

In the third embodiment of the system and method for providing alignment and positioning data of an endoprosthesis fitting of joints, best-fit algorithms are applied in step 5 (S5) before and/or during surgery for the endoprosthesis fitting of joints, which calculate a position and alignment for the femoral and tibial implant based on at least one preselected parameter of the input data from step S2 (S2) and process it into output data in step S3 (S3). Finally, in step S4 (S4), the output data is provided in real time for an alignment and positioning of the femoral and tibial implant that enables the most individualized kinematics of the knee joint for the respective patient.

Furthermore, step 5 (S5) of the third embodiment of the system and method can be extended by step 5a (S5a), which provides that the applied best-fit algorithms over the complete range of motion of the knee joint at least one of the parameters, namely the tension of the knee ligament apparatus, preferably including the patellar tendon, the position of a joint line of the femur and/or tibia connected to the joint, the forces expected to result in the knee implant (femoral and tibial), the pressure expected between the patella implant and the femoral trochlea, the magnitude and direction of the force vector of the patellar tendon, the position of the patella in the trochlea.

Further, in step 5a (S5a), the best-fit algorithms can also take into account results of an intra-operative joint space measurement.

Furthermore, in step 5a (S5a), the best-fit algorithms can also take into account pre-operative kinematics data such as gait analyses.

In summary, it can be said about the system and method according to the present disclosure that the above three embodiments represent exemplary examples, but the individual aspects of the respective embodiments can be combined with each other as desired.

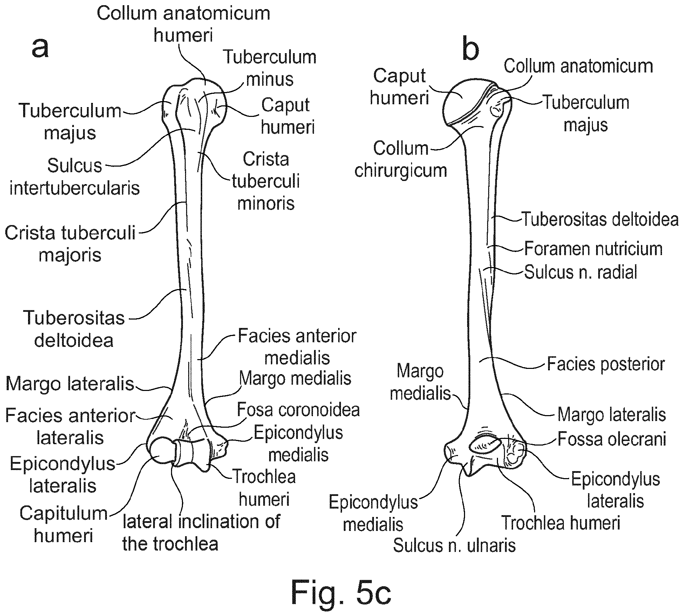

Also, the above-described embodiments of the system and method according to the present disclosure are also applicable to other joints, but especially also for elbow joints. In this specific case, the joint is an elbow joint and the first bone is a humerus (as shown in Fig. 5c) and the second bone is an antebrachium.

Accordingly, best-fit algorithms can also be applied to elbow joints before and/or during surgery, which calculate a position and alignment for the humeral and antebrachial implant based on at least one preselected parameter of the input data and process it into output data, which are subsequently provided in real time as output data for the alignment and positioning of the humeral and antebrachial implant, whereby the provided alignment and positioning enable the most individually suitable kinematics for the respective patient's elbow joint.

In summary the present disclosure refers to a system and method for providing alignment and positioning data of an endoprosthesis fitting of joints, performing the steps of:
- acquiring information as input data on the position and course of the native trochlea of a first bone in relation to the joint centre, the joint line of the first bone and the joint line of a second bone forming the joint together with the first bone, and the bony axes of the first and second bones;
- processing the acquired input data and calculating output data to provide alignment and positioning data of a first joint partial implant of the first bone having an artificial trochlea and a second joint partial implant of the second bone, wherein the artificial trochlea of the first joint partial implant is to replace the native trochlea of the first bone as identically as possible in alignment and position;
- providing the output data as alignment and positioning data.

## Claims

1. A method for providing alignment and positioning data of an endoprosthesis fitting of joints with the steps:
- acquiring information as input data on the position and course of the native trochlea of a first bone in relation to the joint centre, to the joint line of the first bone and to the joint line of a second bone forming the joint together with the first bone, and to the bony axes of the first and second bones;
- processing the acquired input data and calculating output data to provide alignment and positioning data of a first joint partial implant of the first bone having an artificial trochlea and a second joint partial implant of the second bone, wherein the artificial trochlea of the first joint partial implant is to replace the native trochlea of the first bone in alignment and position as identically as possible;
- providing the output data as alignment and positioning data.

2. The method for providing alignment and positioning data of an endoprosthesis fitting of joints according to claim 1, **characterized in that** the input data refers to a knee joint according to which the first bone is a femur with a femoral trochlea and the second bone is a tibia.

3. The method for providing alignment and positioning data of an endoprosthesis fitting of joints according to claim 2, **characterized by** the steps of:
- simultaneously considering measured input data of a patellar ligament tension during alignment of the tibia;
- processing and matching the patellar ligament tension input data with reference values;
- providing output data for tibial alignment based on predetermined target tolerance ranges of ligament stress.

4. The method for providing alignment and positioning data of an endoprosthesis fitting of joints according to any one of claims 2 or 3, **characterized by** the steps of:
- applying best-fit algorithms before and/or during surgery to calculate a position and alignment for the femoral and tibial implant based on at least one preselected parameter of the input data and processing the same into output data;
- real-time provision of the output data for the alignment and positioning of the femoral and tibial implant, wherein the provided alignment and positioning enable the individual best possible kinematics of the knee joint for the respective patient.

5. The method for providing alignment and positioning data of an endoprosthesis fitting of joints according to claim 4, **characterized in that** the best-fit algorithms over the complete range of motion of the knee joint include at least one of the parameters,
- tension of the knee ligament apparatus, preferably including the patellar tendon;
- position of a joint line of the femur and/ or tibia connected to the joint;
- forces expected to result in the knee implant;
- pressure expected between the patella implant and the femoral trochlea;
- magnitude and direction of the patellar tendon force vector;
- position of the patella in the trochlea.

6. The method for providing alignment and positioning data of an endoprosthesis fitting of joints according to claim 4 or 5, **characterized in that** results of an intra-operative joint gap measurement are taken into account in the best-fit algorithms.

7. The method for providing alignment and positioning data of an endoprosthesis fitting of joints according to one of claims 4 to 6, **characterized in that** pre-operative kinematic data, preferably also in the form of gait analyses, are taken into account in the best-fit algorithms.

8. The method for providing alignment and positioning data of an endoprosthesis fitting of joints according to any one of claims 4 to 7, **characterized in that** the best-fit algorithms take into account and process the influence of changes to the alignment and orientation of the implant components in the course of a pre-operative and/or intra-operative planning and/or implementation in the best-fit calculations in the form of input data in real time and subsequently reproduce the effects on the individual aspects as output data.

9. The method for providing alignment and positioning data of an endoprosthesis fitting of joints according to claim 1, **characterized in that** the joint is an elbow joint and the first bone is a humerus and the second bone is an antebrachium.

10. The method of providing alignment and positioning data of an endoprosthesis fitting of joints according to claim 9, **characterized by** the steps of:
- applying best-fit algorithms before and/or during surgery to calculate a position and alignment for the humeral and antebrachial implant based on at least one preselected parameter of the input data and processing the same into output data;
- real-time provision of the output data for the alignment and the positioning of the humeral and antebrachial implant, wherein the provided alignment and positioning enable the individual best possible kinematics of the elbow joint for the respective patient.

11. A system adapted to provide alignment and positioning data of an endoprosthesis fitting of joints comprising
- an acquiring unit (1), being adapted to acquire information as input data on the position and course of the native trochlea of a first bone in relation to the joint centre, to the joint line of the first bone and to the joint line of a second bone forming the joint together with the first bone, and to the bony axes of the first and second bones;
- a processing unit (2), being adapted to process the acquired input data and calculating output data to provide alignment and positioning data of a first joint partial implant of the first bone having an artificial trochlea and a second joint partial implant of the second bone, wherein the artificial trochlea of the first joint partial implant is to replace the native trochlea of the first bone in alignment and position as identically as possible and
- a provisioning unit (3) being adapted to provide the output data as alignment and positioning data.

12. The system according to claim 11, **characterized in that** the input data refers to a knee joint according to which the first bone is a femur with a femoral trochlea and the second bone is a tibia.

13. The system to claim 12, **characterized in that** the acquiring unit (1) is adapted to simultaneously consider measured input data of a patellar ligament tension during alignment of the tibia and the processing unit (2) is adapted to process and match the patellar ligament tension input data with reference values wherein the provisioning unit (3) is adapted to provide output data for tibial alignment based on predetermined target tolerance ranges of ligament stress.

14. The system according to any one of claims 12 or 13, **characterized in that** the processing unit (2) is adapted to apply best-fit algorithms before and/or during surgery to calculate a position and alignment for the femoral and tibial implant based on at least one preselected parameter of the input data and processing the same into output data wherein the provisioning unit (3) is adapted for real-time provision of the output data for the alignment and positioning of the femoral and tibial implant, wherein the provided alignment and positioning enable the individual best possible kinematics of the knee joint for the respective patient.

15. The system according to claim 14, **characterized in that** the best-fit algorithms over the complete range of motion of the knee joint include at least one of the parameters,
- tension of the knee ligament apparatus, preferably including the patellar tendon;
- position of a joint line of the femur and/ or tibia connected to the joint;
- forces expected to result in the knee implant;
- pressure expected between the patella implant and the femoral trochlea;
- magnitude and direction of the patellar tendon force vector;
- position of the patella in the trochlea.

16. The system according to claim 14 or 15, **characterized in that** results of an intra-operative joint gap measurement are taken into account in the best-fit algorithms.

17. The system according to one of claims 14 to 16, **characterized in that** pre-operative kinematic data, preferably also in the form of gait analyses, are taken into account in the best-fit algorithms.

18. The system according to any one of claims 14 to 17, **characterized in that** the best-fit algorithms take into account and process the influence of changes to the alignment and orientation of the implant components in the course of a pre-operative and/or intra-operative planning and/or implementation in the best-fit calculations in the form of input data in real time and subsequently reproduce the effects on the individual aspects as output data.

19. The system according to claim 1, **characterized in that** the input data refers an elbow joint wherein the first bone is a humerus and the second bone is an antebrachium.

20. The system according to claim 19, **characterized in that**
- the processing unit (2) is adapted to apply best-fit algorithms before and/or during surgery to calculate a position and alignment for the humeral and antebrachial implant based on at least one preselected parameter of the input data and processing the same into output data and
- the provisioning unit (3) is adapted for real-time provision of the output data for the alignment and the positioning of the humeral and antebrachial implant, wherein the provided alignment and positioning enable the individual best possible kinematics of the elbow joint for the respective patient.
